# EUROPEAN PATENT SPECIFICATION

(11) **EP 1 167 973 B1**
(45) Date of publication and mention of the grant of the patent: **14.04.2010**
(21) Application number: 01902760.6
(22) Date of filing: 05.02.2001
(51) Int. Cl.: G01N 33/52

(54) **CHROMATOGRAPHY SPECIMEN AND METHOD FOR PREPARATION THEREOF**
CHROMATOGRAPHISCHE PROBE UND VERFAHREN ZU IHRER HERSTELLUNG
DISPOSITIF DE CHROMATOGRAPHIE ET PROCEDE DE PREPARATION CORRESPONDANT

(30) Priority: 04.02.2000 JP 2000027988
(43) Date of publication of application: 02.01.2002
(73) Proprietor: Panasonic Corporation, Kadoma-shi Osaka 571-8501 (JP)
(72) Inventor: TAKAHASHI, Mie, Niihama-shi Ehime 792-0026 (JP); Nadoaka, Masataka, Iyo-shi, Ehime 799-3113 (JP); Tanaka, Hirotaka, Matsuyama-shi, Ehime 791-1102 (JP)
(74) Representative: Balsters, Robert
(86) International application number: PCT/JP2001/000784
(87) International publication number: WO 2001/057531

(56) References cited:
- EP-A- 0 162 301
- EP-A- 0 605 394
- EP-A- 0 889 327
- EP-A- 0 903 584
- EP-A- 1 473 342
- JP-A- 3 120 468
- JP-A- 3 120 469
- JP-A- 3 120 470
- JP-A- 11 064 336
- JP-A- 11 153 601
- US-A- 4 258 001
- US-A- 5 558 834
- US-A- 5 976 823
- TECHNICAL BULLETIN BASF, 2002, pages 1-1, XP002312662 USA
- TECHNICAL BULLETIN, 2002, pages 1-1, XP002312663
- DATABASE FAO [Online] FAO; FAO: "Polyoxyethylene (40) Stearate" retrieved from HTTP://WWW.FAO.ORG/AG/AGN/JECFA-ADDITIVES/ SPECS/MONOGRAPH1/ADDITIVE-325.PDF
- DATABASE PAN ASIA CHEMICAL CORP [Online] PAN ASIA CHEMICAL CORP; PAN ASIA CHEMICAL CORP: "Polyethylene Glycol Ester" retrieved from HTTP://WWW.PACCT.COM.TW/SPECIFICATION/N1/P EG_ESTER.PDF
- DATABASE SURFACTANT [Online] SURFACTANT; SURFACTANT: "POLYOXYETHYLENE ALKYL (and ALKYL ARYL) ETHER" retrieved from HTTP://WWW.SURFACTANT.CO.KR/SURFACTANTS/PO E.HTML

## Description

### TECHNICAL FIELD

The present invention relates to a chromatography specimen for qualitatively or quantitatively analyzing a liquid sample and its manufacturing method and, more particularly, to a specimen on which a liquid sample spreads uniformly.

### BACKGROUND ART

Conventionally, a measuring method by chromatography, which utilizes an antigen-antibody reaction or enzyme reaction is generally used as a method for implementing a chemical test for liquid samples such as examination of water and urinalysis, or a clinical test. Usually, the chromatography is a method for separating a mixture according to its components.

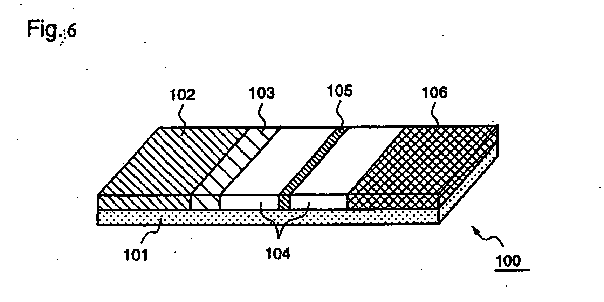

Figure 6 is a diagram illustrating a conventional chromatography specimen which is used for measurement by the chromatography.

In figure 6, a chromatography specimen 100 has a reactive layer carrier support body 101 which supports a chromatography material, a sample application part 102 to which a liquid sample is applied, a marker hold region 103 which holds a marker reagent which can be moved by permeation of the liquid sample, a reactive layer 104 in which a binding reaction is processed between the marker reagent having a substance that is specifically bound to an analysis target included in a liquid sample which flows therein and the analysis target, a specific protein immobilization part 105 in which a specific protein that specifically processes a binding reaction with an analysis target such as an antibody and an antigen according to a reaction format is immobilized on the region of the reactive layer 104, and a water-absorbing part 106 for absorbing the liquid sample which flows therein.

Next, a measuring method using the conventional chromatography specimen 100 will be described.

When a liquid sample is applied to the sample application part 102, the liquid sample permeates the sample application part 102 and reaches to the marker hold region 103. Then, a marker reagent held in the area of the marker hold region 103 is dissolved due to the permeation of the liquid sample and permeates the reactive layer 104 with the liquid sample. On the region of the reactive layer 104, there is the specific protein immobilization part 105 in which a specific protein is immobilized, and when the liquid sample includes an analysis target, the specific protein processes an antigen-antibody reaction with a complex of the analysis target and the marker reagent, and some color reaction is seen in the region of the specific protein immobilization part 105. This color reaction is measured visually or by adopting a detection device. On the other hand, when the liquid sample does not include an analysis target, no antigen-antibody reaction is processed nor no color reaction is seen. The liquid sample is finally absorbed into the water-absorbing part 106, and the reaction is ended. As described above, in the measuring method using the chromatography specimen, since the result of the test is shown in the color reaction and thus its judgement is quite easy, it is widely applicable and further can be utilized for test of various analysis targets. However, the measurement using the chromatography specimen makes a liquid sample spread on the specimen and confirms the color reaction. Therefore, a more uniform spread pattern of the liquid sample on the specimen is required for obtaining an accurate measurement result, and further a reaction between the marker reagent and the liquid sample on the reactive layer 104 is affected by the spread speed of the liquid sample, whereby an improvement in the permeation of the liquid sample to the reactive layer 104 is also required.

Conventional examples of the chromatography specimen are disclosed for example in Japanese Published Patent Application No. Sho.62-71861 and No. Hei.11-153601

Japanese Published Patent Application No. Sho.62-718.61 discloses a method by which a surface active agent having a HLB (hydrophile-lipophile balance) value which is larger than 20 is applied to promote a reaction, thereby detecting an analysis target speedily, in measurement employing immunity principles. Further, the Japanese Published Patent Application No. Hei.11-153601 discloses a method by which an additive impregnating part which carries a surface active agent or the like is provided between the sample application part and the reactive layer, and a decrease in a S/N ratio and an erroneous operation due to the coloring of a background and the generation of a blank is prevented, whereby an analysis target is detected accurately and speedily.

However, for the chromatography specimen, there is conventionally no means for mechanically controlling a permeation speed of a liquid sample, and thus it is impossible to artificially control the permeation speed, whereby the permeation speed of the liquid sample depends on the permeability of the specimen. Thus, a considerable time may be required till the liquid sample permeates the specimen, the permeation of the liquid sample to the reactive layer 104 may not be uniform, or a reactive component immobilization part such as the specific protein immobilization part 105 may have a part which is not permeated by the sample, thereby lacking precision in measurement. To solve these problems, a surface active agent processing is executed to the specimen to enhance the permeation performance of the chromatography specimen. However, when a surface active agent whose original property is in liquid or paste form is used as the surface active agent to be adopted for the surface active agent processing, it is impossible to dry the surface active agent up to an absolute dry condition. Thereby, an immobilized antibody is gradually devitalized during a conservation period of the chromatography specimen and the performance of the specimen is deteriorated, whereby a quality maintenance period of the specimen is shortened or a storage condition of the specimen is restricted.

In the chromatography measurement, a liquid sample is applied on a specimen, and a color area where a color reaction is processed a prescribed period of time after the application of the liquid sample is measured. Therefore, when the specimen is subjected to a surface active agent processing, a marker reagent processes a nonspecific adsorption to the reactive layer 104 and the marker reagent remains on the reactive layer as a background, and thus the value of the background is added to an essential degree of the coloring to include an error when the color area is detected by adopting a detecting instrument, whereby a quantitative performance is reduced. Also when the color reaction is visually judged, the color of the background leads to an erroneous recognition in the judgement of the essential color situation.

When the method disclosed in the Japanese Published Patent Application No. Sho.62-71861 is adopted, a surface active agent having a HLB value which is higher than 20 is applied to increase the permeability of the specimen. Therefore, the permeability and the permeation speed of the specimen is surely increased, while the permeation speed is too high to perform a sufficient reaction required for the measurement, resulting in the lack of the precision in measurement.

Further, when the measurement is performed by adopting the chromatography specimen as disclosed in the Japanese Published Patent Application No. Hei.11-153601, since the surface active agent is impregnated on a region situated forward the reactive layer on the specimen, the surface active agent does not exist in a reactive component immobilization part such as the specific protein immobilization part, whereby influences of the background can be reduced and reactive components of the specimen are not devitalized nor denatured. However, the sample does not sufficiently permeate the reactive components in the immobilization area in a stage where the permeation is developed, whereby the reaction is not performed uniformly, resulting in the lack of precision in measurement.

The present invention is made to solve the above-mentioned problems and has for its object to provide a chromatography specimen and its manufacturing method which minimize the quantity of a marker reagent remaining in the background, enhance the reactivity by improvement in the permeability of a liquid sample and a more uniform spread of the liquid sample, and enhance the preservation stability of the chromatography specimen.

EP-A-0 162 301 discloses an integral multilayer chromatography specimen comprising a porous spreading layer provided on a reagent layer or water absorbing layer, wherein said porous spreading layer includes an hydrophilic cellulose derivative and a non-ionic surfactant having an HLB value of not less than 10 such as ethyleneoxide addition compounds of polyhydric alcohol esters (condensates) such as sorbitan tristearate, polyethylene glycol monoesters, polyethylene glycol diesters, ethyleneoxide addition compounds of higher alcohols (condensates), ethyleneoxide addition compounds of alkylphenols (condensates) and alkanolamides of higher fatty acids.

EP-A-0 889 327 discloses a lateral flow-type chromatographic specimen which comprises a wicking pad (a sample application part), a GSA pad (a marker hold region) and a reactive layer, wherein said GSA pad includes a surfactant such as surfactants available under the tradename Tetronic ® which are tetrafunctional block copolymers of derived from the sequential addition of propylene oxide and ethylene oxide to ethylenediamine.

### DISCLOSURE OF THE INVENTION

According to Claim 1 of the present invention, there is provided a chromatography specimen which is a flow through-type chromatography specimen obtained by laminating a plurality of wettable porous materials or a lateral flow type chromatography specimen comprising a single-layer porous material, said chromatography specimen comprising : a sample application part to which a liquid sample is added or applied; a marker hold region for holding a marker reagent so that it can be dissolved in the liquid sample; a reactive layer having, in its region, a part to which at least one of reactive components to be used for chromatograph analysis is immobilized; and a water absorbing part for finally absorbing the liquid sample; wherein said reactive layer includes a surface active agent having such a property that it can be solidified when dried, and wherein said surface active agent comprises a sugar in its hydrophilic part.

Therefore, the reactivity of the chromatography specimen can be enhanced due to the enhancement in the permeability of the reactive layer and the uniform permeation of a sample, thereby realizing a chromatography measurement with a higher sensitivity and a higher performance. Further, by adopting the surface active agent having such a property that it can be solidified when dried as the surface active agent, the devitalization of a reactive component immobilized on the reactive layer can be minimized, thereby realizing the enhanced preservation stability, the extended quality maintenance period, and the expanded storage condition of the chromatography specimen.

Further, the solubility is enhanced and the permeability is increased by the action of sugar, while influence upon protein can be reduced, whereby the denaturation or devitalization of immobilized specific protein can be minimized and thus the performance of the reactive layer can be held for a long time.

According to Claim 2 of the present invention, in the chromatography specimen as defined in Claim 1, the surface active agent comprises a surface active agent having a HLB (lipophile-hydrophile balance) value which is 20 or lower.

Therefore, in addition to the effect according to Claim 1, it can be prevented that the permeation speed of a liquid sample in the reactive layer is too high to obtain a sufficient reaction, and further by selecting the HLB value to control the reaction speed appropriately, a chromatography measurement with a higher sensitivity and a higher performance can be realized.

According to Claim 3 of the present invention, in the chromatography specimen as defined in Claim 1 or 2, the reactive layer includes the surface active agent in the entirety thereof.

Therefore, the reactivity of the chromatography specimen can be enhanced due to the enhancement in the permeability of the reactive layer and the uniform permeation of a sample, thereby realizing a chromatography measurement with a higher sensitivity and a higher performance. Further, by adopting the surface active agent having such a property that it can be solidified when dried as the surface active agent, the devitalization of a reactive component immobilized on the reactive layer can be minimized, thereby realizing the enhanced preservation stability, the extended quality maintenance period, and the expanded storage condition of the chromatography specimen.

According to Claim 4 of the present invention, in the chromatography specimen as defined in any of Claims 1 to 3, the reactive layer includes the surface active agent in a part thereof.

Therefore, the reactivity of the chromatography specimen can be enhanced due to the enhancement in the permeability of the reactive layer and the uniform permeation of a sample, thereby realizing a chromatography measurement with a higher sensitivity and a higher performance. Further, by adopting the surface active agent having such a property that it can be solidified when dried as the surface active agent, the devitalization of a reactive component immobilized on the reactive layer can be minimized, thereby realizing the enhanced preservation stability, the extended quality maintenance period, and the expanded storage condition of the chromatography specimen.

According to Claim 5 of the present invention, there is provided a method for manufacturing a chromatography specimen which is a flow through-type chromatography specimen obtained by laminating a plurality of wettable porous materials or a lateral flow type chromatography specimen comprising a single-layer porous material, said chromatography specimen having a sample application part to which a liquid sample is added or applied, a marker hold region for holding a marker reagent so that it can be dissolved in the liquid sample, a reactive layer having, in its region, a part to which at least one of reactive components to be used for chromatograph analysis is immobilized, and a water-absorbing part for finally absorbing the liquid sample, said method comprising : a step of impregnating or coating the reactive layer with a surface active agent dissolved liquid in which a surface active agent having such a property that it can be solidified when dried is dissolved; and a step of drying the surface active agent dissolved liquid with which the reactive layer has been impregnated or coated, wherein the surface active agent comprises a sugar in its hydrophilic part.

Therefore, the reactivity of the chromatography specimen can be enhanced due to the enhancement in the permeability of the reactive layer and the uniform permeation of a sample, whereby a chromatography specimen with a higher sensitivity and a higher performance can be manufactured. Further, by adopting the surface active agent having such a property that it can be solidified when dried as the surface active agent, the devitalization of a reactive component immobilized on the reactive layer can be minimized, whereby the chromatography specimen having the enhanced preservation stability, the extended quality maintenance period, and the expanded storage condition can be manufactured.

Further, in addition to the above effect, the solubility is enhanced and the permeability is increased by the action of sugar, while influence upon protein can be reduced, whereby the denaturation or devitalization of immobilized specific protein can be minimized and thus the chromatography specimen whose performance of the reactive layer can be held for a long time can be manufactured.

According to Claim 6 of the present invention, in the chromatography specimen manufacturing method as defined in Claim 5, the surface active agent comprises a surface active agent having a HLB (lipophile-hydrophile balance) value which is 20 or lower.

Therefore, in addition to the effect according to Claim 5, it can be prevented that the permeation speed of a liquid sample in the reactive layer is too high to obtain a sufficient reaction, and further by selecting the HLB value to control the reaction speed appropriately, a chromatography specimen with a higher sensitivity and a higher performance can be manufactured.

According to Claim 7 of the present invention, in the chromatography specimen manufacturing method as defined in Claim 5 or 6, the reactive layer is dried by air drying.

Therefore, in addition to the effect according to Claim 5, the load to a reactive component immobilized on the reactive layer can be suppressed, thereby manufacturing a chromatography specimen having the performance of the processed reactive layer which can be held for a long time.

According to Claim 8 of the present invention, in the chromatography specimen manufacturing method as defined in any of Claims 5 to 7 the reactive layer is dried by wind drying.

Therefore, in addition to the effect according to Claim 5, the drying time can be shorten and the devitalization or denaturation of an immobilized reactive component during the drying can be minimized, thereby manufacturing a chromatography specimen having the performance of the processed reactive layer which can be held for a long time.

According to Claim 9 of the present invention, in the chromatography specimen manufacturing method as defined in any of Claims 5 to 8 the reactive layer is dried by freeze drying.

Therefore, in addition to the effect according to Claim 5, the property of an immobilized reactive component can be almost held, thereby manufacturing a chromatography specimen having the performance of the processed reactive layer which can be held for a long time.

According to Claim 10 of the present invention, in the chromatography specimen manufacturing method as defined in any of Claims 5 to 9, the entire reactive layer is impregnated or coated with the surface active agent dissolved liquid.

Therefore, the reactivity of the chromatography specimen can be enhanced due to the enhancement in the permeability of the reactive layer and the uniform permeation of a sample, whereby a chromatography specimen with a higher sensitivity and a higher performance can be manufactured. Further, by adopting the surface active agent having such a property that it can be solidified when dried as the surface active agent, the devitalization of a reactive component immobilized on the reactive layer can be minimized, whereby the chromatography specimen having the enhanced preservation stability, the extended quality maintenance period, and the expanded storage condition can be manufactured.

According to Claim 11 of the present invention, in the chromatography specimen manufacturing method as defined in any of Claims 5 to 10, a part of the reactive layer is impregnated or coated with the surface active agent dissolved liquid.

Therefore, the reactivity of the chromatography specimen can be enhanced due to the enhancement in the permeability of the reactive layer and the uniform permeation of a sample, whereby a chromatography specimen with a higher sensitivity and a higher performance can be manufactured. Further, by adopting the surface active agent having such a property that it can be solidified when dried as the surface active agent, the devitalization of a reactive component immobilized on the reactive layer can be minimized, whereby the chromatography specimen having the enhanced preservation stability, the extended quality maintenance period, and the expanded storage condition can be manufactured.

### BRIEF DESCRIPTION OF DRAWINGS

Figure 1 is a perspective view illustrating a structure of a lateral flow-type chromatography specimen according to a first embodiment of the present invention.
Figure 2 is a perspective view illustrating a structure of a flow through-type chromatography specimen according to a second embodiment of the present invention.
Figure 3 is a perspective view illustrating the flow through-type chromatography specimen according to the second embodiment of the invention.
Figure 4 is a perspective view illustrating the flow through-type chromatography specimen according to the second embodiment of the invention.
Figures 5 are diagrams illustrating quantitative performances according to the embodiments of the present invention, figure 5(a) showing a case where a surface active agent is not adopted as a processing for a reactive layer and figure 5(b) showing a case where the surface active agent is adopted.
Figure 6 is a perspective view illustrating a structure of a conventional chromatography specimen.

### BEST MODE TO EXECUTE THE INVENTION

Hereinafter, embodiments according to the present invention will be described with reference to the drawings. The embodiments described here are given only as examples and the present invention is not restricted to these embodiments.

### (Embodiment 1)

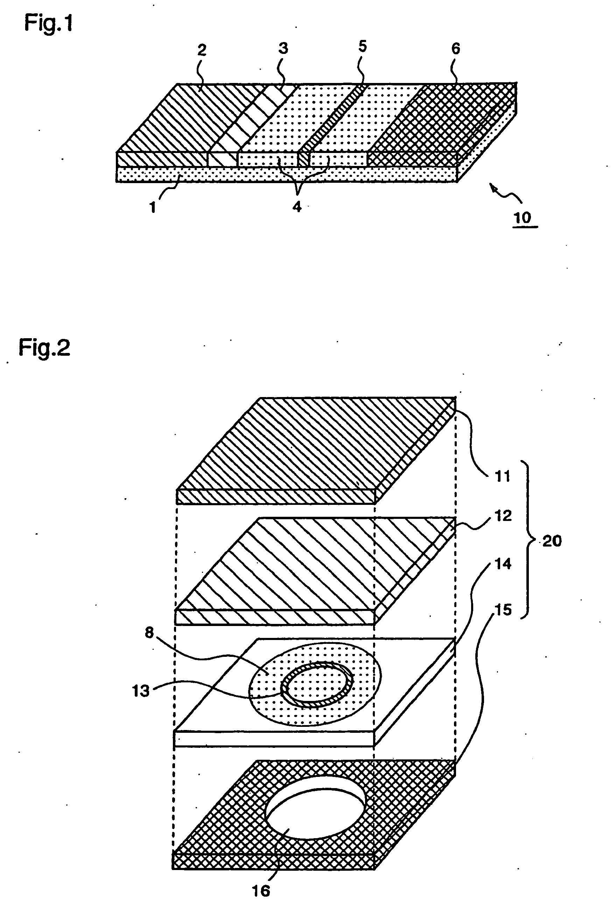

Figure 1 is a diagram illustrating a lateral flow-type chromatography specimen made of a wettable single-layer porous material according to a first embodiment of the present invention.

In figure 1, a lateral flow-type chromatography specimen 10 has a reactive layer carrier support 1, a sample application part 2, a marker hold region 3, a reactive layer 4, a specific protein immobilization part 5, and a water-absorbing part 6.

The reactive layer carrier support 1 is made of liquid-impermeable plastic or the like, and supports a chromatography material. The sample application part 2 is made of a nonwoven fabric having high hydrophilia or the like, and a liquid sample is added or applied thereto. The marker hold region 3 holds a marker reagent on the nonwoven fabric or the like so that it can be dissolved by the liquid sample. The reactive layer 4 is made of nitrocellulose or the like, and further impregnated with a surface active agent dissolved liquid in which a surface active agent having such a property that it can be solidified when dried is dissolved and thereafter dried. In the present invention, the surface active agent is the general term for substances which include a hydrophilic atomic group comprising sugar and having a high affinity with water molecules and a hydrophobic atomic group having a low affinity with water molecules in its molecule, and have properties of changing properties on the interface or surface. Further, among those surface active agents, the surface active agent having such a property that it can be solidified when dried is the one which can be massed or have a solid shape such as granules and powder when the surface active agent in a high concentration is subjected to a vacuum or freeze drying or to a drying operation at the normal pressures with the addition of heat or at the ordinary temperatures and normal pressures. Further, the impregnation process to the reactive layer 4 is a process for dipping the reactive layer 4 in the surface active agent dissolved liquid. The specific protein immobilization part 5 is obtained by immobilization a specific protein that specifically processes a binding reaction with an analysis target like an antibody or an antigen according to the reaction format on the region of the reactive layer 4. The water-absorbing part 6 finally absorbs the liquid sample. The sample application part 2, the marker hold region 3, the reactive layer 4 having the specific protein immobilization part 5, and the water-absorbing part 6 are put on the top of the reactive layer carrier support 1, thereby forming the lateral flow-type chromatography specimen 10.

The marker reagent held in the marker hold region 3 and the specific protein immobilized on the specific immobilization part 5 on the chromatography specimen 10 should be selected properly according to a sample to be analyzed and an analysis target.

As the surface active agent dissolved liquid with which the reactive layer 4 is impregnated, the surface active agent having sugar in its hydrophilic part and having such a property that it is solidified when dried preferably has a HLB value which is 20 or lower.

Here, when more preferable surface active agents which are used in the surface active agent dissolved liquid are listed, as for the one including the surface active agent having a HLB value which is 20 or lower, one including a surface active agent that has a HLB value which is near 20 and has a structure in which many hydrophilic atomic groups are included is more preferable. The surface active agent having sugar in its hydrophilic part includes preferably a surface active agent having a structure in which a sugar chain such as Sucrose Monolaurate and n-Octyl-β-D-Thioglucoside is included. The above-described names of substances are merely examples.

While the surface active agent processing to the reactive layer 4 is performed by the impregnation processing which impregnates the reactive layer 4 with the surface active agent dissolved liquid, it may be performed by a coating processing which coats the reactive layer 4 with the surface active agent dissolved liquid. The surface active agent processing to the reactive layer 4 can be performed either to a part or the whole of the reactive layer 4.

As the processing for drying the surface active agent dissolved liquid with which the reactive layer 4 has been impregnated, there are processings such as air drying which is a method of leaving it at ordinary temperatures and normal pressures to be dried, wind drying which is a method of applying a prescribed wind force to it at an arbitrary temperature to be dried, and freeze drying.

A chromatography material which is composed of an arbitrary porous carrier such as nitrocellulose and glass fiber filter paper is adopted as the lateral flow-type chromatography specimen 10.

Next, a chromatographic analysis method which adopts the lateral flow-type chromatography specimen 10 according to the first embodiment will be described.

In figure 1, when a liquid sample is applied to the sample application part 2, the liquid sample permeates the sample application part 2 and reaches to the marker hold region 3. Then, a marker reagent held in the area of the marker hold region 3 is dissolved due to the permeation of the liquid sample and permeates the reactive layer 4 together with the liquid sample. Then, a surface active agent included in the reactive layer 4 is dissolved with the permeation of the liquid sample. By the action of the dissolved surface active agent, permeation into the reactive layer 4 is speedily performed and the permeation of the liquid sample is proceeded with the end of the permeating liquid being lined and without remaining. In the area of the reactive layer 4, there is the specific protein immobilization part 5 on which a specific protein is immobilized, and when the liquid sample includes an analysis target, the specific protein processes an antigen-antibody reaction with a complex of the analysis target and the marker reagent, resulting in some color reaction in the area of the specific protein immobilization part 5. When the liquid sample does not include an analysis target, the antigen-antibody reaction is not processed and no color reaction is seen. Finally, the liquid sample is absorbed by the water-absorbing part 6 and the reaction is ended.

As described above, according to the lateral flow-type chromatography specimen 10 of the first embodiment, the permeability of the chromatography specimen 10 is enhanced and a more uniform permeation is enabled by the processing for impregnating the reactive layer 4 on the chromatography specimen 10 with the surface active agent dissolved liquid and the drying processing. This enhancement in permeability and the uniform permeation of the liquid sample improve the reactivity on the chromatography specimen 10, resulting in a chromatography measurement with a higher sensitivity and a higher performance.

When the surface active agents used for the surface active agent dissolved liquid with which the reactive layer 4 is impregnated include the surface active agent having such a property that it can be solidified when dried, the reactive layer 4 is in a completely dried condition until the liquid sample is applied thereto and permeates the reactive layer 4. Therefore, devitalization of the immobilized specific protein can be minimized, resulting in the enhancement in preservation stability, the extension of the quality maintenance period, and the relaxation of storage conditions on the chromatography specimen 10.

In addition, when the surface active agent comprising the surface active agent which has a HLB value which is 20 or lower is adopted, it can be avoided that the permeation speed of the liquid sample into the reactive layer 4 becomes too high to obtain a sufficient reaction. Further, when the HLB value is selected to adjust its reaction speed appropriately, a chromatography measurement with a higher sensitivity and a higher performance can be realized.

When the surface active agent comprising the surface active agent which has sugar in its hydrophilic part is adopted, the solubility is increased and the permeability is enhanced by the action of sugar, while the denaturation or devitalization of the immobilized specific protein can be minimized since the influence upon protein is a little, whereby the performance of the reactive layer 4 can be held for a long time.

When the reactive layer 4 is dried by the air drying, the load onto the specific protein immobilized on the reactive layer 4 can be suppressed, whereby the performance of the specimen 10 can be held for a long time.

When the reactive layer 4 is dried by the wind drying, the drying time can be shorten and the devitalization or denaturation of the specific protein during the drying can be minimized, whereby the performance of the specimen 10 can be held for a long time.

When the reactive layer 4 is dried by the freeze drying, the properties of the specific protein can be almost held, whereby the performance of the specimen 10 can be held for a long time.

### (Embodiment 2)

Hereinafter, a flow through-type chromatography specimen according to a second embodiment of the present invention will be described with reference to figures 2, 3 and 4.

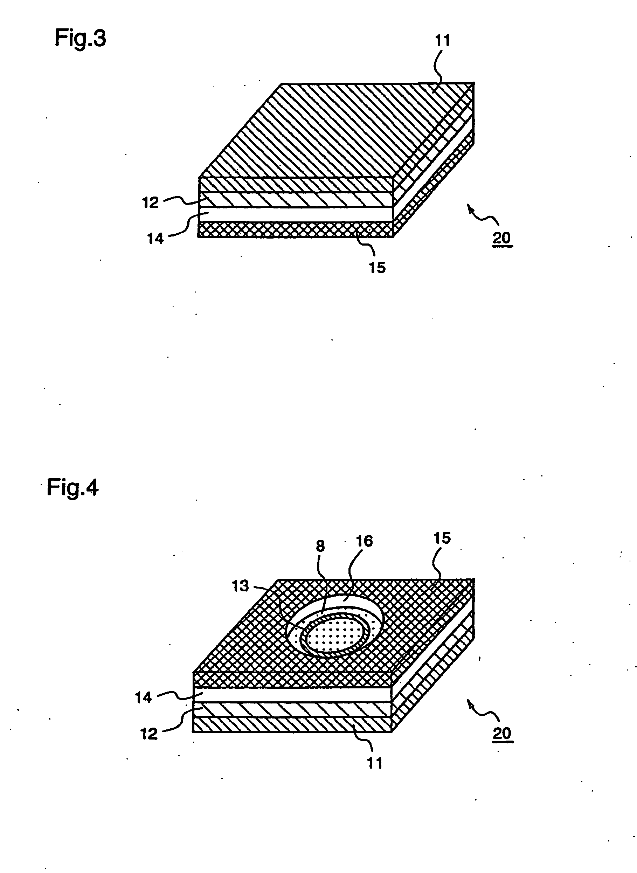

Figure 2 is a perspective view illustrating a structure of a flow through-type chromatography specimen which is constituted by laminating plural wettable porous materials according to the second embodiment. Figure 3 is a perspective view illustrating the flow through-type chromatography specimen seen from the side of a sample application part. Figure 4 is a perspective view of the flow through-type chromatography specimen seen from the side of a water-absorbing part for finally absorbing a liquid sample.

In figure 2, the flow through-type chromatography specimen 20 has a sample application part 11, a marker hold region 12, a surface active processing part 8, a specific protein immobilization part 13, a reactive layer 14, and a water-absorbing part 15.

The sample application part 11 is made of a nonwoven fabric having a high hydrophilia or the like, and a liquid sample is added or applied thereto. The marker hold region 12 holds a marker reagent in the nonwoven fabric or the like so that it can be dissolved. The reactive layer 14 is made of nitrocellulose or the like and has the surface active processing part 8 and the immobilization part 13 on its area. The surface active processing part 8 is obtained by coating a surface active agent dissolved liquid in which a surface active agent having such a property that it can be solidified when dried is dissolved on the reactive layer 14 and then drying the same. The surface active agent shown here and the surface active agent having such a property that it can be solidified when dried are those that are described above in the first embodiment. The specific protein immobilization part 13 is obtained by immobilization a specific protein that specifically processes a binding reaction with an analysis target such as an antibody or antigen according to the reaction format on the area of the surface active processing part 8 of the reactive layer 104. The water-absorbing part 15 has a result confirmation window 16 for seeing the result of the reaction on the reactive layer 14 and finally absorbs the liquid sample. The sample application part 11, the marker hold region 12, the reactive layer 14 including the specific protein immobilization part 13 and the surface active processing part 8, and the water-absorbing part 15 are laminated, thereby forming the flow through-type chromatography specimen 20.

A marker reagent held on the marker hold region 12 and specific protein immobilized on the specific protein immobilization part 13 on the flow through-type chromatography specimen 20 should be selected appropriately according to a sample to be analyzed and an analysis target.

While a surface active agent processing to the reactive layer 14 is performed by the coating processing for coating the reactive layer 14 with the surface active agent dissolved liquid, it may be performed by the impregnation processing for impregnating the reactive layer 14 with the surface active agent dissolved liquid as in the first embodiment. The surface active agent processing may be performed either to a part of the reactive layer 4 as in the second embodiment or to the whole reactive layer as in the first embodiment.

The surface active agent included in the surface active agent dissolved liquid with which the reactive layer 14 is coated, the drying processing performed after the reactive layer 14 is coated with the surface active agent dissolved liquid, and the material of the flow through-type chromatography specimen 20 are the same as those in the first embodiment, and thus their descriptions will be omitted here.

Next, a chromatographic analysis method which adopts the flow through-type chromatography specimen 20 according to the second embodiment will be described.

On the flow through-type chromatography specimen 20 shown in figures 2, 3 and 4, when a liquid sample is applied to the sample application part 11, the liquid sample permeates the sample application part 11 and reaches to the marker hold region 12. Then, a marker reagent held in the area of the marker hold region 12 is dissolved due to the permeation of the liquid sample and permeates the reactive layer 14 together with the liquid sample. Then, a surface active agent included in the surface active processing part 8 is dissolved with the permeation of the liquid sample. By the action of the dissolved surface active agent, permeation into the reactive layer 14 is speedily performed and the permeation of the liquid sample is proceeded with the end of the permeating liquid being relatively uniformed and without remaining. On the area of the reactive layer 14, there is the specific protein immobilization part 13 on which a specific protein is immobilized, and when the liquid sample includes an analysis target, the specific protein processes an antigen-antibody reaction with a complex of the analysis target and the marker reagent, resulting in some color reaction in the area of the specific protein immobilization part 13. The color reaction can be seen through the result confirmation window 16 provided in the water-absorbing part 15. When the liquid sample does not include an analysis target, the antigen-antibody reaction is not processed and no color reaction is shown. The liquid sample is finally absorbed into the water-absorbing part 15, and then the reaction is ended.

As described above, according to the flow through-type chromatography specimen 20 of the second embodiment, the permeability of the reactive layer 14 is enhanced and a more uniform permeation is enabled by the impregnation processing and the drying processing of the surface active agent dissolved liquid on the flow through-type chromatography specimen 20. The enhancement in the permeability and the uniform permeation improves the reactivity of the chromatography specimen 20, resulting in a chromatography measurement with a higher sensitivity and a higher performance.

When the surface active agent used for the surface active agent dissolved liquid with which the reactive layer 14 is coated include the surface active agent having such a property that it can be solidified when dried is adopted, the reactive layer 14 is in a completely dried condition till the liquid sample is applied thereto and permeates the reactive layer 14. Therefore, devitalization of the immobilized specific protein can be minimized, resulting in the enhanced preservation stability, the extended quality maintenance period, and the expanded storage condition of the chromatography specimen 20.

When the surface active agent comprising the surface active agent having a HLB value which is 20 or lower is adopted, it can be avoided that the permeation speed of the liquid sample in the reactive layer 14 becomes too high to obtain a sufficient reaction. Further, when the HLB value is selected to control its reaction speed appropriately, a chromatography measurement with a higher sensitivity and a higher performance can be realized.

When the surface active agent comprising the surface active agent having sugar in its hydrophilic part is adopted, the solubility is enhanced and the permeability is increased by the action of sugar, while the denaturation or devitalization of the immobilized specific protein can be minimized since the influence upon protein is a little, whereby the performance of the surface active processing part 8 can be held for a long time.

When the reactive layer 14 is dried by the air drying, the load to the specific protein immobilized on the reactive layer 14 can be suppressed, whereby the performance of the specimen 20 can be held for a long time.

When the reactive layer 14 is dried by wind drying, the drying time can be shorten and the deactivation or denaturation of the specific protein during the drying can.be minimized, whereby the performance of the specimen 20 can be held for a long time.

When the reactive layer 14 is dried by freeze drying, the property of the specific protein can be almost held, whereby the performance of the specimen 20 can be held for a long time.

### (Examples)

A method for implementing the present invention will be described in more detail through following examples.

### (Quantitative analysis of hCG in blood plasma)

A lateral flow-type immunochromatography specimen which includes an anti-hCG-β antibody immobilization line and a broad band of a complex of an anti-hCG-α antibody and gold colloid in a nitrocellulose film is manufactured. This specimen is shown in figure 1. In this figure, the specimen 10 holds the specific protein immobilization part 5 as the anti-hCG-β antibody immobilization line; and the marker hold region 3 forward thereof, which is an area including the complex of the anti-hCG-α antibody and the gold colloid on the reactive layer 4 as a nitrocellulose film, and includes the sample application part 2 made of a nonwoven fabric and the water-absorbing part 6 made of glass fiber filter paper forward and backward thereof, respectively. The specimen 10 is manufactured as follows.

### (Example 1)

### Preparation of lateral flow-type immunochromatography specimen

An anti-hCG-β antibody solution which was diluted with a phosphate buffer solution to control the concentration was prepared. This antibody solution was applied on the nitrocellulose film by adopting a solution discharge device. Thereby, a detecting antibody immobilization line was obtained on the nitrocellulose film. After being dried, this nitrocellulose film was immersed in a Tris-HCl buffer solution including 1% skim milk and shaken gently for 30 minutes. 30 minutes later, the nitrocellulose film was moved into a Tris-HCl buffer solution tank, shaken gently for 10 minutes, and thereafter shaken gently in another Tris-HCl buffer solution tank for another 10 minutes, to wash the nitrocellulose film. After washed twice, the nitrocellulose film was immersed in a Tris-HCl buffer solution including 0.05% Sucrose Monolaurate (Dojindo made), shaken for 10 minutes, then taken out from the solution tank, and dried at room temperatures. Accordingly, the specific protein immobilization part 5 was obtained on the nitrocellulose film as the reactive layer 4.

The gold colloid was prepared by adding 1% citric acid solution to a refluxing 100°C-solution of 0.01% chloroauric acid. After the reflux was continued for 30 minutes, it was cooled. The anti-hCG-α antibody was added to gold colloid solution which was prepared to pH9 by using 0.2M potassium carbonate solution, then the obtained solution was stirred for several minutes, and then 10% BSA (bovine serum albumin) solution pH9 was added thereto by such an amount that 1% solution was finally obtained and stirred. Thereby, an antibody-gold colloid complex (marker antibody) was prepared. The marker antibody solution was centrifuged at 4°C and 20000G for 50 minutes, whereby the marker antibody was isolated, and the isolated marker antibody was suspended in a washing buffer solution (1% BSA · phosphate buffer solution) and thereafter centrifuged to wash and isolate the marker antibody. After suspended in the washing buffer solution and filtrated through a 0.8µm filter, the marker antibody was prepared one-tenth as much as the initial gold colloid solution and stored at 4°C.

The marker antibody solution was set in the solution discharge device and applied to a position on an anti-hCG-β antibody immobilization dry film, apart from an antibody immobilization position, and thereafter the film was dried. Thereby, the marker antibody hold region 3 was obtained on the immobilization film.

The antibody immobilization film including the marker antibody hold region 3 prepared as described above was affixed on the reactive layer carrier support 1, a nonwoven fabric was added thereto as the sample application part 2, glass fiber filter paper was added thereto as the water-absorbing part 6, and thereafter the film was cut into small pieces of 0.5cm width, thereby manufacturing the specimen 10.

### (Example 2)

### Preparation of sample

Human blood to which heparin was added as an anticoagulant was centrifuged at 4000 rpm for 5 minutes to prepare blood plasma in which blood cells were separated. The hCG solutions of known concentrations were added to the plasma, thereby preparing the hCG solutions of various known concentrations.

### (Example 3)

### Measurement of the degree of coloration on immunochromatography specimen

More than 200µl of plasma including hCG was applied to the sample application part 1 on the specimen 10 and spread in the direction of the water-absorbing part 6, to process an antigen-antibody reaction, whereby a color reaction in the specific protein immobilization part 5 on which the anti-hCG-β antibody was immobilized was processed. The coloration state 5 minutes after the sample application to the specimen 10 was measured by adopting a reflective spectrophotometer (CS9300; Shimadzu Corporation made), and the coloration degree is computed.

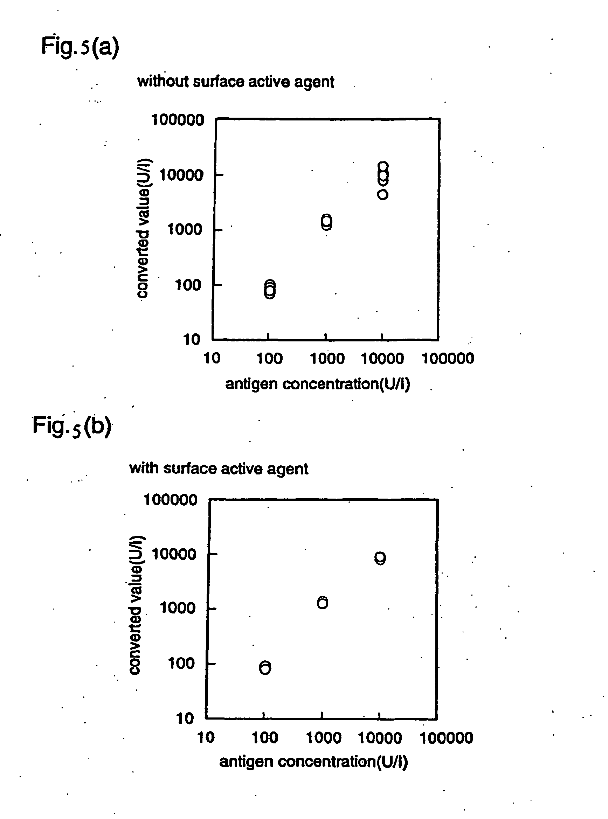

In this example, plasmas including hCG of 0, 100, 1000, and 10000U/1 were applied to the specimen 10 to be spread, and the coloration state of the antibody immobilization part 5 on the specimen 10 for plasma of each hCG concentration was measured by the reflective spectrophotometer. An absorbance at the wavelength of 520nm was measured by the reflective spectrophotometer, and substituted into a previously formed calibration curve indicating a relationship between the hCG concentration and the absorbance. The result is shown in figure 5. Essentially, when for example blood including hCG of 1000U/1 was applied onto each specimen 10, the absorbance of the color area of each specimen 10 was measured, and the measured absorbance in each specimen 10 was substituted into the calibration curve to obtain the hCG concentration, its value should be all 1000U/1. However, a value actually obtained by the substitution into the calibration curve slightly deviates from 1000U/1 according to the reaction condition in each specimen 10. That is, the accuracy of measurement in each specimen 10 can be known by the amount of the deviation.

Figures 5 are diagrams illustrating quantitative performances in the immunochromatography specimen formed as described above, figure 5(a) showing a case where the reactive layer is not processed with a surface active agent and figure 5(b) showing a case where it is processed with the surface active agent. The abscissa represents the hCG concentration of a sample applied to the specimen 10. The ordinate represents the converted value of the antigen concentration obtained by substituting the absorbance from a marker in the color area on the specimen 10 into the calibration curve.

Hereinafter, descriptions will be given of effects in a case where the reactive layer 4 is not processed with a surface active agent and a case where it has been processed with the surface active agent in the immunochromatography specimen 10. The reactive layer which has been processed with a surface active agent shown here is one that is washed twice in a Tris-HCl buffer solution, then immersed in a Tris-HCl buffer solution including 0.05% Sucrose Monolaurate and shaken gently for 10 minutes, and thereafter dried by leaving the same at room temperatures, as shown in the Example 1. The reactive layer which is not processed indicates one that is washed twice in the Tris-HCl buffer solution and dried by leaving the same at room temperature.

Figures 5 show the result obtained by converting the concentration of an analysis target on the basis of a measured value of a coloration degree, five minutes after a liquid sample is applied to the immunochromatography specimen 10. The marker reagent used at this time is the same antibody-gold colloid complex both in figures 5(a) and 5(b). In a case where the reactive layer 4 has been processed with a surface active agent (figure 5(b)), a CV value (coefficient of variation) is within 0 to 7%, while in a case where it is not processed witch a surface active agent (figure 5(a)), the CV value ranges from 15 to 35% having a wide range of variations, and has a low quantitative performance. From the above results, it can be understood that the use of the reactive layer which has been impregnated with the surface active agent in the immunochromatography specimen 10 greatly concerns the enhancement in the quantitative performance. While in this example the comparison description has been given with respect to the quantitative performance, a more accurate result is obtained also in a qualitative test when a chromatography specimen having a reactive layer which is impregnated with a surface active agent is adopted.

Since the gold colloid is adopted as the marker in this example, the coloration degree with a wavelength of 520nm is measured. However, an absorbance with any wavelength may be measured as long as it is an absorption wavelength of the marker.

### APPLICABILITY IN INDUSTRY

A chromatography specimen and its manufacturing method according to the present invention are significantly useful as one which enhances the permeation speed and the permeability in a reactive layer of the chromatography specimen, uniforms the permeability, and enhance the measurement efficiency in the chromatography specimen.

## Claims

1. A chromatography specimen which is a flow through-type chromatography specimen obtained by laminating a plurality of wettable porous materials or a lateral flow type chromatography specimen comprising a single-layer porous material, said chromatography specimen comprising :
a sample application part (2, 11) to which a liquid sample is to be added or applied;
a marker hold region (3, 12) for holding a marker reagent so that it can be dissolved in the liquid sample;
a reactive layer (4, 14) having, in its region, a part (5, 13) to which at least one of reactive components to be used for chromatograph analysis is immobilized; and
a water absorbing part (6, 15) for finally absorbing the liquid sample;
wherein said reactive layer includes a surface active agent having such a property that it can be solidified when dried, and
wherein said surface active agent comprises a sugar in its hydrophilic part.

2. The chromatography specimen as defined in Claim 1, wherein the surface active agent has a HLB (lipophile-hydrophile balance) value which is 20 or lower.

3. The chromatography specimen as defined in Claims 1 or 2,
wherein the reactive layer includes the surface active agent in the entirety thereof.

4. The chromatography specimen as defined in claims 1 or 2,
wherein the reactive layer includes the surface active agent in a part thereof.

5. A method for manufacturing a chromatography specimen which is a flow through-type chromatography specimen obtained by laminating a plurality of wettable porous materials or a lateral flow type chromatography specimen comprising a single-layer porous material, said chromatography specimen having a sample application part (2, 11) to which a liquid sample is added or applied, a marker hold region (3, 12) for holding a marker reagent so that it can be dissolved in the liquid sample, a reactive layer (4, 14) having, in its region, a part (5, 13) to which at least one of reactive components to be used for chromatograph analysis is immobilized, and a water-absorbing part (6, 15) for finally absorbing the liquid sample,
said method comprising:
a step of impregnating or coating the reactive layer with a surface active agent dissolved liquid in which a surface active agent having such a property that it can be solidified when dried is dissolved; and
a step of drying the surface active agent dissolved liquid with which the reactive layer has been impregnated or coated,
wherein the surface active agent comprises a sugar in its hydrophilic part.

6. The chromatography specimen manufacturing method as defined in Claim 5,
wherein the surface active agent has a HLB (lipophile-hydrophile balance) value which is 20 or lower.

7. The chromatography specimen manufacturing method as defined in Claims 5 or 6,
wherein the reactive layer is dried by air drying.

8. The chromatography specimen manufacturing method as defined in Claims 5 or 6,
wherein the reactive layer is dried by wind drying.

9. The chromatography specimen manufacturing method as defined in Claims 5 or 6,
wherein the reactive layer is dried by freeze drying.

10. The chromatography specimen manufacturing method as defined in any of Claims 5 to 9,
wherein the entire reactive layer is impregnated or coated with the surface active agent dissolved liquid.

11. The chromatography specimen manufacturing method as defined in any of Claims 5 to 9,
wherein a part of the reactive layer is impregnated or coated with the surface active agent dissolved liquid.

## Patentansprüche

1. Chromatographischer Probekörper, der ein Durchfluss-chromatographischer Probekörper, der durch Aufeinanderschichten einer Vielzahl von benetzbaren porösen Materialien erhalten wird, oder ein Lateralfluss-chromatographischer Probekörper aus einem einschichtigen porösen Material ist, mit:
einem Probenaufgabeteil (2, 11), auf den eine flüssige Probe gegeben oder aufgebracht werden soll;
einem Markierungsreagens-Haltebereich (3, 12) zum Halten eines Markierungsreagens so, dass es in der flüssigen Probe gelöst werden kann;
einer reaktionsfähigen Schicht (4, 14), die in ihrem Bereich einen Teil (5, 13) hat, auf dem mindestens eine von mehreren reaktionsfähigen Komponenten, die für eine chromatographische Analyse verwendet werden sollen, immobilisiert wird; und
einem wasseraufnehmenden Teil (6, 15) zum abschließenden Aufnehmen der flüssigen Probe,
wobei
die reaktionsfähige Schicht einen oberflächenaktiven Stoff hat, der so beschaffen ist, dass er beim Trocknen verfestigt werden kann, und
der oberflächenaktive Stoff in seinem hydrophilen Teil einen Zucker enthält.

2. Chromatographischer Probekörper nach Anspruch 1, **dadurch gekennzeichnet, dass** der oberflächenaktive Stoff einen HLB-Wert (HLB: hydrophilic-lipophilic balance; Hydrophil-Lipophil-Gleichgewicht) hat, der gleich oder kleiner als 20 ist.

3. Chromatographischer Probekörper nach Anspruch 1 oder 2, **dadurch gekennzeichnet, dass** die reaktionsfähige Schicht den oberflächenaktiven Stoff in der gesamten reaktionsfähigen Schicht enthält.

4. Chromatographischer Probekörper nach Anspruch 1 oder 2, **dadurch gekennzeichnet, dass** die reaktionsfähige Schicht den oberflächenaktiven Stoff in einem Teil der reaktionsfähigen Schicht enthält.

5. Verfahren zur Herstellung eines chromatographischen Probekörpers, der ein Durchfluss-chromatographischer Probekörper, der durch Aufeinanderschichten einer Vielzahl von benetzbaren porösen Materialien erhalten wird, oder ein Lateralfluss-chromatographischer Probekörper aus einem einschichtigen porösen Material ist, wobei der chromatographische Probekörper Folgendes aufweist:
einen Probenaufgabeteil (2, 11), auf den eine flüssige Probe gegeben oder aufgebracht wird;
einen Markierungsreagens-Haltebereich (3, 12) zum Halten eines Markierungsreagens so, dass es in der flüssigen Probe gelöst werden kann;
eine reaktionsfähige Schicht (4, 14), die in ihrem Bereich einen Teil (5, 13) hat, auf dem mindestens eine von mehreren reaktionsfähigen Komponenten, die für eine chromatographische Analyse verwendet werden sollen, immobilisiert wird; und
einen wasseraufnehmenden Teil (6, 15) zum abschließenden Aufnehmen der flüssigen Probe,
wobei das Verfahren die folgenden Schritte aufweist:
einen Schritt des Imprägnierens oder Beschichtens der reaktionsfähigen Schicht mit einer Flüssigkeit, in der ein oberflächenaktiver Stoff gelöst ist, der so beschaffen ist, dass er beim Trocknen verfestigt werden kann; und
einen Schritt des Trocknens der Flüssigkeit, in der der oberflächenaktive Stoff gelöst ist und mit der die reaktionsfähige Schicht imprägniert oder beschichtet worden ist,
wobei der oberflächenaktive Stoff in seinem hydrophilen Teil einen Zucker enthält.

6. Verfahren zur Herstellung eines chromatographischen Probekörpers nach Anspruch 5, **dadurch gekennzeichnet, dass** der oberflächenaktive Stoff einen HLB-Wert hat, der gleich oder kleiner als 20 ist.

7. Verfahren zur Herstellung eines chromatographischen Probekörpers nach Anspruch 5 oder 6, **dadurch gekennzeichnet, dass** die reaktionsfähige Schicht durch Lufttrocknung getrocknet wird.

8. Verfahren zur Herstellung eines chromatographischen Probekörpers nach Anspruch 5 oder 6, **dadurch gekennzeichnet, dass** die reaktionsfähige Schicht durch Windtrocknung getrocknet wird.

9. Verfahren zur Herstellung eines chromatographischen Probekörpers nach Anspruch 5 oder 6, **dadurch gekennzeichnet, dass** die reaktionsfähige Schicht durch Gefriertrocknung getrocknet wird.

10. Verfahren zur Herstellung eines chromatographischen Probekörpers nach einem der Ansprüche 5 bis 9, **dadurch gekennzeichnet, dass** die gesamte reaktionsfähige Schicht mit der Flüssigkeit, in der der oberflächenaktive Stoff gelöst ist, imprägniert oder beschichtet wird.

11. Verfahren zur Herstellung eines chromatographischen Probekörpers nach einem der Ansprüche 5 bis 9, **dadurch gekennzeichnet, dass** ein Teil der reaktionsfähigen Schicht mit der Flüssigkeit, in der der oberflächenaktive Stoff gelöst ist, imprägniert oder beschichtet wird.

## Revendications

1. Spécimen pour chromatographie qui est un spécimen pour chromatographie de type à flux continu obtenu par stratification d'une pluralité de matières poreuses mouillables ou un spécimen pour chromatographie de type à flux latéral comprenant une matière poreuse en couche unique, ledit spécimen pour chromatographie comprenant :
une partie d'application d'échantillon (2, 11) à laquelle un échantillon liquide doit être ajouté ou appliqué ;
une partie de maintien de marqueur (3, 12) pour maintenir un réactif marqueur de façon à ce qu'il puisse être dissous dans l'échantillon liquide ;
une couche réactive (4, 14) ayant, dans sa région, une partie (5, 13) sur laquelle au moins l'un de composants réactifs devant être utilisé pour une analyse par chromatographe est immobilisé ; et
une partie d'absorption d'eau (6, 15) pour finalement absorber l'échantillon liquide ;
dans lequel ladite couche réactive inclut un agent actif en surface ayant une propriété telle qu'il peut être solidifié lorsqu'il est séché, et
dans lequel ledit agent actif en surface comprend un sucre dans sa partie hydrophile.

2. Spécimen pour chromatographie selon la revendication 1,
dans lequel l'agent actif en surface a une valeur HLB (équilibre lipophile-hydrophile) qui est de 20 ou moins.

3. Spécimen pour chromatographie selon les revendications 1 ou 2,
dans lequel la couche réactive inclut l'agent actif en surface dans la totalité de celle-ci.

4. Spécimen pour chromatographie selon les revendications 1 ou 2,
dans lequel la couche réactive inclut l'agent actif en surface dans une partie de celle-ci.

5. Procédé de fabrication d'un spécimen pour chromatographie qui est un spécimen pour chromatographie de type à flux continu obtenu par stratification d'une pluralité de matières poreuses mouillables ou un spécimen pour chromatographie de type à flux latéral comprenant une matière poreuse en couche unique, ledit spécimen pour chromatographie ayant une partie d'application d'échantillon (2, 11) à laquelle un échantillon liquide est ajouté ou appliqué, une partie de maintien de marqueur (3, 12) pour maintenir un réactif marqueur de façon à ce qu'il puisse être dissous dans l'échantillon liquide, une couche réactive (4, 14) ayant, dans sa région, une partie (5, 13) sur laquelle au moins l'un de composants réactifs devant être utilisé pour une analyse par chromatographe est immobilisé, et une partie d'absorption d'eau (6, 15) pour finalement absorber l'échantillon liquide,
ledit procédé comprenant :
une étape d'imprégnation ou de revêtement de la couche réactive avec un liquide dissous d'agent actif en surface dans laquelle un agent actif en surface ayant une propriété telle qu'il peut être solidifié lorsqu'il est séché est dissous ; et
une étape de séchage du liquide dissous d'agent actif en surface avec lequel la couche réactive a été imprégnée ou revêtue,
dans lequel ledit agent actif en surface comprend un sucre dans sa partie hydrophile.

6. Procédé de fabrication d'un spécimen pour chromatographie selon la revendication 5,
dans lequel l'agent actif en surface a une valeur HLB (équilibre lipophile-hydrophile) qui est de 20 ou moins.

7. Procédé de fabrication d'un spécimen pour chromatographie selon les revendications 5 ou 6,
dans lequel la couche réactive est séchée par séchage à l'air.

8. Procédé de fabrication d'un spécimen pour chromatographie selon les revendications 5 ou 6,
dans lequel la couche réactive est séchée par séchage à l'air pulsé.

9. Procédé de fabrication d'un spécimen pour chromatographie selon les revendications 5 ou 6,
dans lequel la couche réactive est séchée par lyophilisation.

10. Procédé de fabrication d'un spécimen pour chromatographie selon l'une quelconque des revendications 5 à 9,
dans lequel la totalité de la couche réactive est imprégnée ou revêtue avec le liquide dissous d'agent actif en surface.

11. Procédé de fabrication d'un spécimen pour chromatographie selon l'une quelconque des revendications 5 à 9, '
dans lequel une partie de la couche réactive est imprégnée ou revêtue avec le liquide dissous d'agent actif en surface.
